# EUROPEAN PATENT APPLICATION

(11) **EP 4 241 703 A1**
(43) Date of publication of application: **13.09.2023**
(21) Application number: 21889650.4
(22) Date of filing: 08.11.2021
(51) Int. Cl.: A61B 17/32, A61B 17/11, A61F 5/00, A61B 5/07, A61B 34/20, A61B 34/00, A61B 17/34

(54) **SLEEVE GASTRECTOMY ASSISTING DEVICE**

(30) Priority: 09.11.2020 KR 20200148872
(71) Applicant: Ajou University Industry-Academic Cooperation Foundation, Gyeonggi-do 16499 (KR); Soonchunhyang University Industry Academy Cooperation Foundation, Asan-si, Chungcheongnam-do 31538 (KR)
(72) Inventor: LEE, Moon Gu, Suwon-si, Gyeonggi-do 16499 (KR); KIM, Sang Hyun, Seoul 05287 (KR); YUN, Sang Chul, Seoul 04069 (KR); CHO, Sung Woo, Seoul 05502 (KR); KANG, Young Jae, Gwangju 62250 (KR)
(74) Representative: Zacco Sweden AB
(86) International application number: PCT/KR2021/016116
(87) International publication number: WO 2022/098185

(57) **Abstract**

The present disclosure relates to a sleeve gastrectomy assisting device including a resection guide configured to be inserted into a stomach of a patient and have a changeable form, a measurer configured to be installed at the resection guide and measure a pressure applied to the resection guide, and a monitor configured to be connected to the measurer and provide at least one of a resection length information or a resection position information through a pressure value measured by the measurer.

## Description

### [Technical Field]

The present disclosure relates to a sleeve gastrectomy assisting device, and more particularly, to a sleeve gastrectomy assisting device capable of being inserted into the stomach to assist the resection surgery.

### [Background Art]

Generally, sleeve gastrectomy is a typical surgical method for severe obesity. Sleeve gastrectomy is a laparoscopic surgery in which the stomach is cut using special tools such as a camera for observation, a stapler, and a traction device, leaving only a thin, constant-diameter stomach with a volume of 100 to 150 ml. Unlike other surgeries, sleeve gastrectomy is surgery to simply reduce the size of the stomach and directly accesses the internal organs without an external incision, thus allowing a quick post-surgery recovery. As compared to other surgical methods, sleeve gastrectomy has advantages that the user's burden is low and the complication rate is the lowest. Sleeve gastrectomy is performed by inserting a bougie into the lumen of the stomach through the mouth and cutting the stomach using a stapler and a traction device according to the form of the bougie.

However, conventional surgical tools for sleeve gastrectomy do not have a means to quantitatively provide necessary information during resection, and thus the process of cutting and pulling the stomach depends on the user's skill without a set standard, and there is a problem that there is a large difference between the form of the stomach remaining after surgery and the target form of the stomach. In a case in which the volume of the remaining stomach is too small, gastroesophageal reflux occurs as a side effect, and in a case in which the volume of the remaining stomach is too large, there is a problem that a patient may fail to reduce weight or the patient's weight may increase again.

The related art of the present disclosure is disclosed in Korean Unexamined Patent Application Publication No. 10-2020-0103489 (Date of Publication: September 2, 2020, Title of Disclosure: Inserting tube for gastric resection guides)

### [Disclosure]

### [Technical Problem]

The present disclosure is directed to providing a sleeve gastrectomy assisting device capable of accurately and promptly providing information on a resection situation.

### [Technical Solution]

The present disclosure provides a sleeve gastrectomy assisting device including: a resection guide configured to be inserted into a stomach of a patient and have a changeable form; a measurer configured to be installed at the resection guide and measure a pressure applied to the resection guide; and a monitor configured to be connected to the measurer and provide at least one of a resection length information or a resection position information through a pressure value measured by the measurer.

Also, the measurer may be provided as a plurality of measurers and disposed in a longitudinal direction of the resection guide, and each of the measurers may independently measure the pressure applied to the resection guide.

Also, the measurer may be made of a flexible material, and the shape of the measurer may be changed according to the form of the resection guide.

Also, the monitor may include: a communication device connected to the measurer to receive the pressure value; a controller configured to determine the at least one of the resection length information or the resection position information based on the pressure value transmitted to the communication device; and a display configured to display the information determined by the controller.

Also, the resection position information may be information on a distance from the resection guide to a resection tool.

Also, the controller may determine resection position sections according to a size of the pressure value measured by the measurer.

Also, the resection position sections may include: an approach-necessary section indicating a case in which the size of the pressure measured by the measurer is lower than or equal to a first set pressure; a separation-necessary section indicating a case in which the size of the pressure measured by the measurer is higher than or equal to a second set pressure; and a resection-possible section indicating that the size of the pressure measured by the measurer is between the first set pressure and the second set pressure.

Also, the resection length information may be information on a length of the resection guide inserted into the stomach.

Also, the controller may calculate a number of measurers measuring an initial pressure inside the stomach.

In addition, the display may display different colors according to the resection position sections.

### [Advantageous Effects]

In a sleeve gastrectomy assisting device according to the present disclosure, since a resection guide is provided to have a freely-changeable form, insertion of the resection guide into the stomach can be smoothly performed, and the form of the stomach remaining after resection can be natural.

Also, in the sleeve gastrectomy assisting device according to the present disclosure, since a measurer is provided as a plurality of measurers and includes a flexible material, the measurer can measure a more accurate pressure value and provide information on a resection length.

Also, in the sleeve gastrectomy assisting device according to the present disclosure, since a controller determines a resection position through pressure inside the stomach that is measured by the measurer, the form of the remaining stomach can be accurately formed regardless of the user's sense.

Also, in the sleeve gastrectomy assisting device according to the present disclosure, since the controller determines a resection length through pressure inside the stomach that is measured by the measurer, it is possible to perform suitable surgery for patients with stomachs of different forms and sizes.

In addition, in the sleeve gastrectomy assisting device according to the present disclosure, since a display displays different colors according to a resection position section, resection position information and resection length information can be provided to be more intuitively identifiable.

### [Description of Drawings]

FIG. 1 is an installation state diagram illustrating an installation state of a sleeve gastrectomy assisting device according to one embodiment of the present disclosure.
FIG. 2 is a perspective view schematically illustrating a configuration of the sleeve gastrectomy assisting device according to one embodiment of the present disclosure.
FIG. 3 is a cross-sectional view illustrating a cross-section of the sleeve gastrectomy assisting device according to one embodiment of the present disclosure.
FIG. 4 is a flowchart schematically illustrating a configuration of a monitor according to one embodiment of the present disclosure.
FIG. 5 is a view schematically illustrating resection position sections determined by a controller according to one embodiment of the present disclosure.
FIGS. 6 to 13 are operation diagrams schematically illustrating operation states of the sleeve gastrectomy assisting device according to one embodiment of the present disclosure.

### [Modes of the Invention]

Hereinafter, embodiments of a sleeve gastrectomy assisting device according to the present disclosure will be described with reference to the accompanying drawings.

In this process, thicknesses of lines or sizes of elements illustrated in the drawings may be exaggerated for clarity and convenience of description. Also, terms used below are terms defined in consideration of functions in the present disclosure and may vary according to an intention or customary practice of a user or an operator. Therefore, the terms should be defined based on the content throughout the specification.

Also, in this specification, when a certain portion is described as being "connected (or linked)" to another portion, this may include not only a case in which the portion is "directly connected (or linked)" to the other portion but also a case in which the portion is "indirectly connected (or linked)" to the other portion while another member is disposed therebetween. In this specification, when a certain portion is described as "including (or having)" a certain element, unless particularly described otherwise, this indicates that the certain portion may further "include (or have)" other elements instead of excluding other elements.

Also, throughout the specification, like reference numerals may refer to like elements. Even when not mentioned or described with reference to specific drawings, like or similar reference numerals may be described based on other drawings. Also, parts not denoted by reference numerals in specific drawings may be described based on other drawings. Also, the number, shape, and size of specific elements, a relative difference between the sizes of the elements, and the like included in the drawings of the present application are set for convenience of understanding and may be implemented in various other forms while not limiting the embodiments.

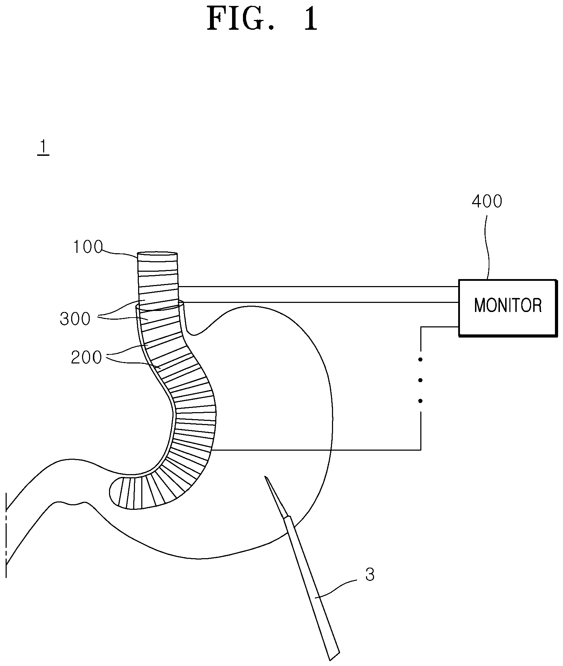

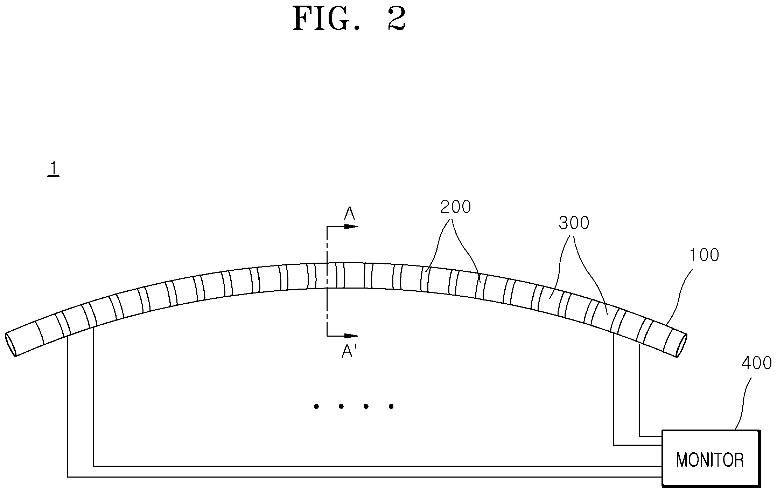

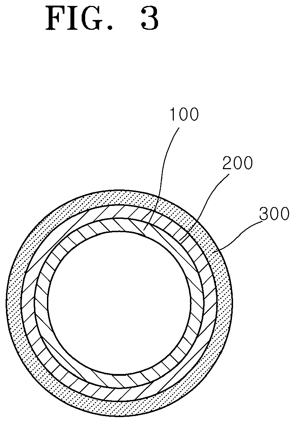

FIG. 1 is an installation state diagram illustrating an installation state of a sleeve gastrectomy assisting device according to one embodiment of the present disclosure, FIG. 2 is a perspective view schematically illustrating a configuration of the sleeve gastrectomy assisting device according to one embodiment of the present disclosure, and FIG. 3 is a cross-sectional view illustrating a cross-section of the sleeve gastrectomy assisting device according to one embodiment of the present disclosure.

Referring to FIGS. 1 to 3, a sleeve gastrectomy assisting device 1 according to one embodiment of the present disclosure includes a resection guide 100, a measurer 200, a packing 300, and a monitor 400.

The resection guide 100 substantially forms an exterior of the sleeve gastrectomy assisting device 1 according to one embodiment of the present disclosure. During sleeve gastrectomy, the resection guide 100 is inserted into a stomach 2 via esophagus through an oral cavity or nasal cavity of a patient. The resection guide 100 occupies a predetermined volume inside the stomach 2, thus distinguishing between an area of the stomach 2 where resection is performed and an area of the stomach 2 where resection is not performed. The resection guide 100 may serve as a reference point of pressure measurement by the measurer 200 which will be described below and thus may guide a resection path of a resection tool 3, which is a stapler, for example.

The resection guide 100 includes a flexible material such as silicone or synthetic resin, and thus the form of the resection guide 100 may be changed. Accordingly, the insertion of the resection guide 100 into the stomach 2 may be more smoothly performed, and by being bent corresponding to the flexure, form, or the like of the stomach, the resection guide 100 may roughly determine the form of the stomach 2 remaining after the resection surgery.

The resection guide 100 according to one embodiment of the present disclosure is formed to have a tubular form including a flexible material such as silicone. A diameter of the resection guide 100 may be formed to have a smaller value than a diameter of the patient's esophagus. The length of the resection guide 100 may be changed to various other values within the range of the length that allows the resection guide 100 to be inserted into the stomach 2. An end of the resection guide 100 is formed to be convex with a predetermined curvature, and thus the insertion of the resection guide 100 into the stomach 2 may be more smoothly performed.

The measurer 200 is installed at the resection guide 100 and measures pressure applied to the resection guide 100. More specifically, during the resection surgery, the measurer 200 measures an internal pressure of the stomach 2 that is applied to the resection guide 100 as the resection tool 3 presses the stomach 2.

The measurer 200 is provided as a plurality of measurers 200, and the plurality of measurers 200 are disposed side by side in a longitudinal direction of the resection guide 100. In this case, each of the measurers 200 independently measures pressure applied to the resection guide 100 in the longitudinal direction of the resection guide 100. Accordingly, the measurers 200 may expand a pressure measurement site and thus measure a more accurate pressure value.

Some of the plurality of measurers 200 are provided to be disposed outside of the stomach 2, that is, inside the esophagus or duodenum, in a state in which the resection guide 100 is completely inserted into the stomach 2. Accordingly, before the resection surgery, as the measurer 200 disposed inside the stomach 2 and the measurer 200 disposed outside the stomach 2 measure different pressure values, a length of the resection guide 100 inserted into the stomach 2 may be converted into the number of measurers 200 measuring an initial pressure P0 inside the stomach, and thus the resection length may be more accurately identified.

The measurer 200 may include a flexible material whose form can be freely changed. Accordingly, since the shape of the measurer 200 can be changed according to the form of the resection guide 100 having flexibility, installation stability can be improved, and pressure applied to the resection guide 100 can be more accurately measured.

The measurer 200 according to one embodiment of the present disclosure may include a flexible circuit board (FCB) having the form of a hollow ring and bent to surround an outer peripheral surface of the resection guide 100 and various types of pressure sensors installed on the FCB to measure a pneumatic pressure. The measurer 200 is provided as about fifteen to twenty measurers 200, and the plurality of measurers 200 are disposed to be spaced apart at predetermined intervals in the longitudinal direction of the resection guide 100. The number of measurers 200 is not limited to the number illustrated in FIGS. 1 to 3 and may be changed within the range of the number of some of the measurers 200 that may be disposed outside the stomach 2 in the state in which the resection guide 100 is completely inserted into the stomach 2.

The packing 300 is disposed outside the resection guide 100 and is provided to surround the measurer 200. The packing 300 may include a flexible material such as silicone or synthetic resin. Accordingly, the packing 300 may firmly fix the measurer 200 to the resection guide 100 while preventing introduction of foreign matter into the measurer 200. The packing 300 according to one embodiment of the present disclosure is formed to have the form of a hollow ring and comes in close contact with outer side surfaces of the measurer 200 and the resection guide 100. A length of the packing 300 may have a greater value than the length of the resection guide 100 at which the measurer 200 is installed. The packing 300 may be made of the same material as the resection guide 100.

The monitor 400 is connected to the measurer 200, determines at least one of a resection length information or a resection position information through a pressure value measured by the measurer 200, and provides the determined information to the user. Here, an example of the resection position information may be information on a distance from the resection guide 100 to the resection tool 3. Also, an example of the resection length information may be information on the length of the resection guide 100 inserted into the stomach.

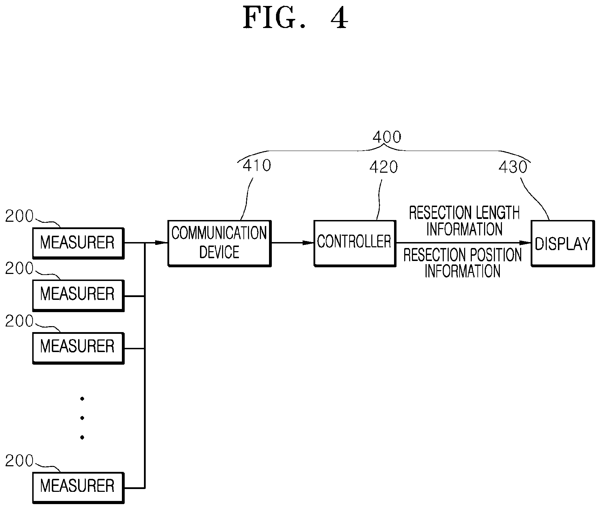

FIG. 4 is a flowchart schematically illustrating a configuration of the monitor according to one embodiment of the present disclosure.

Referring to FIGS. 1 to 4, the monitor 400 according to one embodiment of the present disclosure includes a communication device 410, a controller 420, and a display 430.

The communication device 410 is connected to the measurer 200 to receive a pressure value and transmits the received pressure value to the controller 420 which will be described below. The communication device 410 may transmit the pressure value measured by the measurer 200 to the controller 420 via a wire or wirelessly. The communication device 410 according to one embodiment of the present disclosure connects the measurer 200 and the controller 420, and examples of the communication device 410 may be a signal cable configured to transmit the pressure value via a wire or a communication module configured to wirelessly transmit the pressure value using infrared rays, radio frequency (RF), Bluetooth, or the like.

The controller 420 determines at least one of a resection length information or a resection position information based on the pressure value transmitted to the communication device. Here, as described above, an example of the resection position information may be information on the distance from the resection guide 100 to the resection tool 3, and an example of the resection length information may be information on the length of the resection guide 100 inserted into the stomach. Hereinafter, for convenience of description, the case in which the controller 420 determines both the resection length information and the resection position information will be described as an example.

By determining resection position sections D according to the size of the pressure value measured by the measurer 200, the controller 420 determines the resection position information. By converting the pressure value measured by the measurer 200 into the information on the distance from the resection guide 100 to the resection tool 3, the controller 420 according to one embodiment of the present disclosure determines the resection position information of the resection tool 3.

The resection position sections D are sections obtained by dividing pressure values according to the sizes thereof, based on a value of the initial pressure P0 inside the stomach 2 that is measured by the measurer 200 right after insertion of the resection guide 100. More specifically, the resection position sections D refer to sections between a pair of different pressure values among sections of pressures measured by the measurer 200 as the position at which the stomach 2 is pressed or resected by the resection tool 3 is changed after the insertion of the resection guide 100.

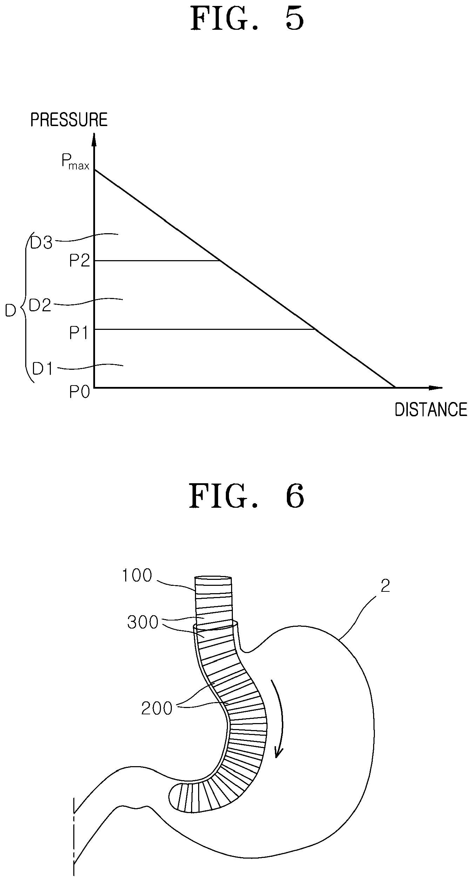

FIG. 5 is a view schematically illustrating the resection position sections determined by the controller according to one embodiment of the present disclosure.

Referring to FIG. 5, the resection position sections D according to one embodiment of the present disclosure include an approach-necessary section D1, a resection-possible section D2, and a separation-necessary section D3.

The approach-necessary section D 1 indicates a case in which the size of the pressure measured by the measurer 200 is the initial pressure P0 or higher and a first set pressure P1 or lower. Here, the first set pressure P1 is a pressure value measured at the maximum distance among distances from the resection guide 100 to the resection tool 3 that allow the user to form the remaining stomach 2 with a target size. The first set pressure P1 may be changed in various ways according to the form, size, and the like of the stomach 2. That is, the approach-necessary section D1 corresponds to a section in which the controller 420 determines, through the pressure value measured by the measurer 200, that the resection tool 3 is excessively far away from the resection guide 100.

The separation-necessary section D3 indicates a case in which the size of the pressure measured by the measurer 200 is a second set pressure P2 or higher and lower than or equal to the maximum pressure that can be measured by the measurer 200 or a pressure Pmax at which rupture of mucosa of the stomach 2 occurs. Here, the second set pressure P2 is a pressure value measured at the minimum distance among the distances from the resection guide 100 to the resection tool 3 that allow the user to form the remaining stomach 2 with a target size. The second set pressure P2 may be changed in various ways according to the form, size, and the like of the stomach 2. That is, the separation-necessary section D3 corresponds to a section in which the controller 420 determines, through the pressure value measured by the measurer 200, that the resection tool 3 is excessively close to the resection guide 100.

The resection-possible section D2 indicates a case in which the size of the pressure measured by the measurer 200 is between the first set pressure P1 and the second set pressure P2. That is, the resection-possible section D2 corresponds to a section in which the controller 420 determines, through the pressure value measured by the measurer 200, that the resection tool 3 is positioned at an appropriate distance from the resection guide 100.

By calculating the number of measurers 200 measuring the initial pressure P0 inside the stomach 2 after the insertion of the resection guide 100, the controller 420 determines the resection length information. In the state in which the resection guide 100 is completely inserted into the stomach 2, the controller 420 according to one embodiment of the present disclosure calculates each of the number of measurers 200 disposed inside the stomach 2 and measuring the same measurement value as the initial pressure P0 inside the stomach 2 measured in advance by a separate measurement device or the like and the number of measurers 200 disposed outside the stomach 2 and measuring a different measurement value from the initial pressure P0. The controller 420 determines that the measurers 200 measuring the same measurement value as the initial pressure P0 inside the stomach 2 are in the approach-necessary section D1 and excludes other measurers 200 from targets for determination.

The display 430 visually displays information determined by the controller 420 to the user. By a display module or the like configured to output image information, the display 430 displays different colors according to the type of resection position section D determined by the controller 420. In a case in which the measurer 200 is provided as a plurality of measurers 200, the display 430 may individually display information on each of the measurers 200.

The display 430 according to one embodiment of the present disclosure displays a first color C1 for the measurers 200 excluded from the targets for determination by the controller 420, displays a second color C2 for the measurers 200 determined as being in the approach-necessary section D1 by the controller 420, displays a third color C3 for the measurers 200 determined as being in the separation-necessary section D3 by the controller 420, and displays a fourth color C4 for the measurers 200 determined as being in the resection-possible section D2 by the controller 420. The first color C 1 to the fourth color C4 may be changed in various ways within the types of colors distinguishable from each other.

Hereinafter, operation states of the sleeve gastrectomy assisting device 1 according to one embodiment of the present disclosure will be described.

FIGS. 6 to 13 are operation diagrams schematically illustrating the operation states of the sleeve gastrectomy assisting device according to one embodiment of the present disclosure.

Referring to FIG. 6, the resection guide 100 inserted through an oral cavity or nasal cavity of a patient is inserted into the stomach 2 via the esophagus. The resection guide 100 inserted into the stomach 2 has an end disposed to face the pylorus of the stomach 2 and a side surface disposed close to an inner side surface of the stomach 2. The shape of the resection guide 100 is changed corresponding to the shape of the inner side surface of the stomach 2, and in this way, the resection guide 100 guides the resection path of the resection tool 3 and roughly determines the form of the stomach 2 remaining after the resection.

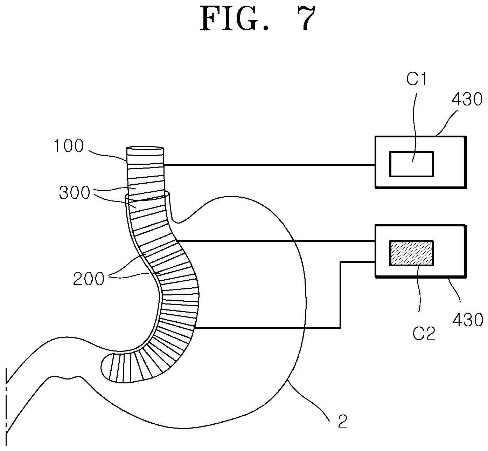

Referring to FIG. 7, the controller 420 determines resection length information through a length of the resection guide 100 inserted into the stomach 2, and the display 430 displays the determined information to the outside.

More specifically, in the state in which the resection guide 100 is completely inserted into the stomach 2, the controller 420 calculates each of the number of measurers 200 disposed inside the stomach 2 and measuring the same measurement value as the initial pressure P0 inside the stomach 2 measured in advance by a separate measurement device or the like and the number of measurers 200 disposed outside the stomach 2 and measuring a different measurement value from the initial pressure P0. Then, the controller 420 determines that the measurers 200 measuring the same measurement value as the initial pressure P0 inside the stomach 2 are in the approach-necessary section D1 and excludes other measurers 200 from targets for determination. For example, when it is assumed that the length of the resection guide 100 at which the measurers 200 are installed is L and a total of twenty measurers 200 are installed, in the case in which the length of the resection guide 100 inserted into the stomach 2 is 4/5L, the controller 420 determines that sixteen of the twenty measurers 200 are in the approach-necessary section D1 and does not perform determination for the remaining four measurers 200. Then, the controller 420 may directly transmit determination information for each of the measurers 200 to the display 430 individually and may convert the number of measurers 200 determined as being in the approach-necessary section D1 into length information of the resection guide 100 and transmit the length information to the display 430.

Then, the display 430 displays the information determined by the controller 420 to the outside. That is, as illustrated in FIG. 6, by displaying the first color C1 for the measurers 200 excluded from the targets for determination by the controller 420 and displaying the second color C2 for the measurers 200 determined as being in the approach-necessary section D1 by the controller 420, the display 430 displays the length of the resection guide 100 subjected to resection. On the other hand, the display 430 may display the length of the resection guide 100 inserted into the stomach 2 using numbers or characters.

Then, the controller 420 determines resection position information of the resection tool 3 through a change in the pressure value measured by the measurer 200, and the display 430 displays the determined information to the outside.

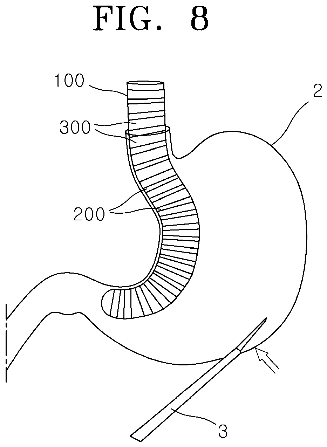

Referring to FIG. 8, in a case in which the resection tool 3 presses the stomach 2 in a state in which the resection tool 3 is further away from the resection guide 100 than the distances from the resection guide 100 to the resection tool 3 that allow the user to form the remaining stomach 2 with a target size, the pressure measured by the measurer 200 is lower than the first set pressure P1.

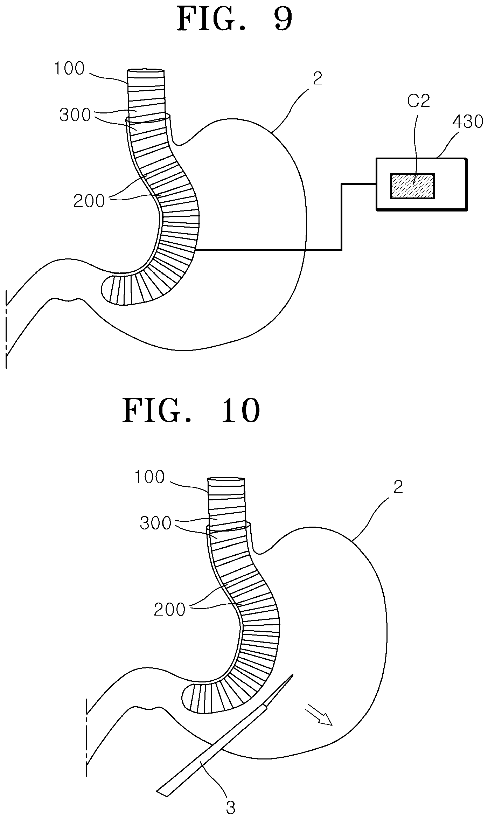

Referring to FIG. 9, the controller 420 determines that the pressure value measured by the measurer 200 is in the approach-necessary section D1 and transmits the corresponding information to the display 430. Then, by displaying the second color C2 to the outside, the display 430 visually provides information indicating that the resection tool 3 is excessively far away from the resection guide 100 and needs to be closer to the resection guide 100.

Referring to FIG. 10, in a case in which the resection tool 3 presses the stomach 2 in a state in which the resection tool 3 is closer to the resection guide 100 than the distances from the resection guide 100 to the resection tool 3 that allow the user to form the remaining stomach 2 with a target size, the pressure measured by the measurer 200 is higher than the second set pressure P2.

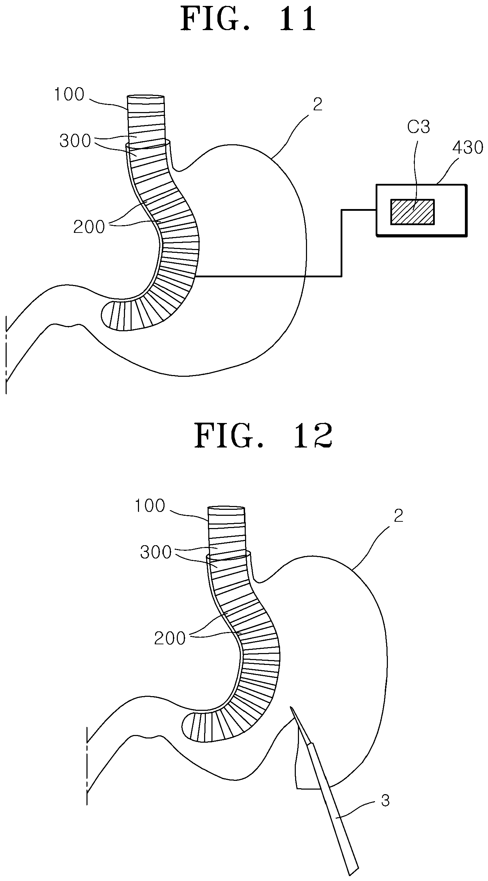

Referring to FIG. 11, the controller 420 determines that the pressure value measured by the measurer 200 is in the separation-necessary section D3 and transmits the corresponding information to the display 430. Then, by displaying the third color C3 to the outside, the display 430 visually provides information indicating that the resection tool 3 is excessively far away from the resection guide 100 and needs to be further from the resection guide 100.

Referring to FIG. 12, in a case in which the resection tool 3 presses the stomach 2 in a state in which the resection tool 3 is placed within the range of the distances from the resection guide 100 to the resection tool 3 that allow the user to form the remaining stomach 2 with a target size, the pressure value measured by the measurer 200 is between the first set pressure P1 and the second set pressure P2.

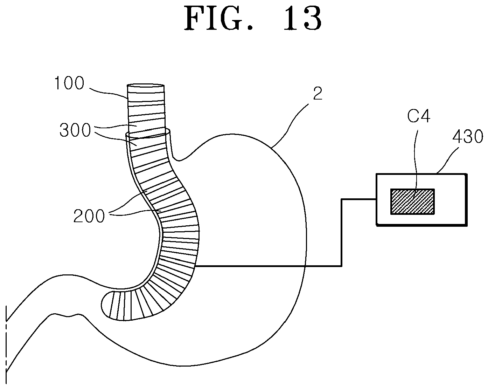

Referring to FIG. 13, the controller 420 determines that the pressure value measured by the measurer 200 is in the resection-possible section D2 and transmits the corresponding information to the display 430. Then, by displaying the fourth color C4 to the outside, the display 430 visually provides information indicating that the resection tool 3 is placed at an appropriate distance from the resection guide 100 and thus resection may be performed using the resection tool 3.

The present disclosure has been described with reference to the embodiments illustrated in the drawings, but the embodiments are merely illustrative, and those of ordinary skill in the art should understand that various modifications and other equivalent embodiments are possible from the embodiments described herein.

Therefore, the technical scope of the present disclosure should be defined by the claims below.

## Claims

1. A sleeve gastrectomy assisting device comprising:
a resection guide configured to be inserted into a stomach of a patient and have a changeable form;
a measurer configured to be installed at the resection guide and measure a pressure applied to the resection guide; and
a monitor configured to be connected to the measurer and provide at least one of a resection length information or a resection position information through a pressure value measured by the measurer.

2. The sleeve gastrectomy assisting device of claim 1, wherein the measurer is provided as a plurality of measurers and disposed in a longitudinal direction of the resection guide, and each of the measurers independently measures the pressure applied to the resection guide.

3. The sleeve gastrectomy assisting device of claim 2, wherein the measurer is made of a flexible material, and a shape of the measurer is changed according to a form of the resection guide.

4. The sleeve gastrectomy assisting device of claim 2, wherein the monitor includes:
a communication device connected to the measurer to receive the pressure value;
a controller configured to determine the at least one of the resection length information or the resection position information based on the pressure value transmitted to the communication device; and
a display configured to display the information determined by the controller.

5. The sleeve gastrectomy assisting device of claim 4, wherein the resection position information is information on a distance from the resection guide to a resection tool.

6. The sleeve gastrectomy assisting device of claim 5, wherein the controller determines resection position sections according to a size of the pressure value measured by the measurer.

7. The sleeve gastrectomy assisting device of claim 6, wherein the resection position sections include:
an approach-necessary section indicating a case in which the size of the pressure measured by the measurer is lower than or equal to a first set pressure;
a separation-necessary section indicating a case in which the size of the pressure measured by the measurer is higher than or equal to a second set pressure; and
a resection-possible section indicating that the size of the pressure measured by the measurer is between the first set pressure and the second set pressure.

8. The sleeve gastrectomy assisting device of claim 4, wherein the resection length information is information on a length of the resection guide inserted into the stomach.

9. The sleeve gastrectomy assisting method of claim 8, wherein the controller calculates a number of measurers measuring an initial pressure inside the stomach.

10. The sleeve gastrectomy assisting device of claim 6, wherein the display displays different colors according to the resection position sections.
